Europäisches Patentamt

European Patent Office

Office européen des brevets

(19)

(11) **EP 1 062 259 B1**

(12) **EUROPÄISCHE PATENTSCHRIFT**

(45) Veröffentlichungstag und Bekanntmachung des Hinweises auf die Patenterteilung:
**15.10.2003  Patentblatt 2003/42**

(51) Int Cl.[7]: **C08G 63/60**

(21) Anmeldenummer: **99908935.2**

(22) Anmeldetag: **20.02.1999**

(86) Internationale Anmeldenummer:
**PCT/EP99/01112**

(87) Internationale Veröffentlichungsnummer:
**WO 99/045054 (10.09.1999 Gazette 1999/36)**

(54) **BIODEGRADABLE POLYMERE AUF DER BASIS VON NATÜRLICHEN UND NACHWACHSENDEN ROHSTOFFEN, INSBESONDERE ISOSORBIT**

BIODEGRADABLE POLYMERS BASED ON NATURAL AND RENEWABLE RAW MATERIALS, ESPECIALLY ISOSORBITE

POLYMERES BIODEGRADABLES A BASE DE MATIERES BRUTES NATURELLES ET RENOUVELABLES, NOTAMMENT D'ISOSORBITOL

(84) Benannte Vertragsstaaten:
**BE DE ES FR GB IT NL**

(30) Priorität: **07.03.1998  DE 19809913**

(43) Veröffentlichungstag der Anmeldung:
**27.12.2000  Patentblatt 2000/52**

(73) Patentinhaber: **Celanese Ventures GmbH 65926 Frankfurt am Main (DE)**

(72) Erfinder:
 • **BENGS, Holger**
   **D-60598 Frankfurt am Main (DE)**
 • **SCHÖNFELD, Axel**
   **D-65207 Wiesbaden (DE)**
 • **BÖHM, Gitte**
   **D-60439 Frankfurt am Main (DE)**
 • **WEIS, Siegfried**
   **D-65817 Eppstein (DE)**
 • **CLAUSS, Joachim**
   **D-60598 Frankfurt am Main (DE)**

(74) Vertreter: **Luderschmidt, Schüler & Partner Patentanwälte Industriepark Höchst 65926 Frankfurt (DE)**

(56) Entgegenhaltungen:
 **EP-A- 0 483 606        EP-A- 0 514 790**

 • **CHEMICAL ABSTRACTS, vol. 127, no. 18, 3. November 1997 Columbus, Ohio, US; abstract no. 248490, OKADA, MASAHIKO ET AL: "Biodegradable polymers based on renewable resources. II. Synthesis and biodegradability of polyesters containing furan rings" XP002103988 & J. POLYM. SCI., PART A: POLYM. CHEM. (1997), 35(13), 2729-2737 CODEN: JPACEC;ISSN: 0887-624X,1997,**
 • **CHEMICAL ABSTRACTS, vol. 123, no. 16, 16. Oktober 1995 Columbus, Ohio, US; abstract no. 199994, OKADA, MASAHIKO ET AL: "Synthesis and biodegradability of polyesters based on 1,4:3,6-dianhydrohexitols and succinic acid derivatives" XP002103989 & STUD. POLYM. SCI. (1994), 12(BIODEGRADABLE PLASTICS AND POLYMERS), 511-18 CODEN: SPLSEA;ISSN: 0922-5579,1994,**
 • **CHEMICAL ABSTRACTS, vol. 124, no. 9, 26. Februar 1996 Columbus, Ohio, US; abstract no. 117772, OKADA, MASAHIKO ET AL: "Synthesis and degradabilities of polyesters from 1,4:3,6-dianhydrohexitol and aliphatic dicarboxylic acids" XP002103990 & J. POLYM. SCI., PART A: POLYM. CHEM. (1995), 33(16), 2813-20 CODEN: JPACEC;ISSN: 0887-624X,1995,**

- **CHEMICAL ABSTRACTS, vol. 126, no. 2, 13. Januar 1997 Columbus, Ohio, US; abstract no. 19395, OKADA, MASAHIKO ET AL: "Biodegradable polymers based on renewable resources: polyesters composed of 1,4: 3,6-dianhydrohexitol and aliphatic dicarboxylic acid units" XP002103991 & J. APPL. POLYM. SCI. (1996), 62(13), 2257-2265 CODEN: JAPNAB;ISSN: 0021-8995, 1996,**

**Beschreibung**

Die Erfindung betrifft biologisch abbaubare Polykondensate auf der Basis von Dianhydrohexiten, deren Herstellung sowie deren Verwendung.

Biologisch abbaubare Polykondensate zeichnen sich dadurch aus, daß sie in einem biologischen Milieu innerhalb einer bestimmten Zeitspanne abgebaut werden. Dabei verlieren die Polykondensate ihre anfänglichen Eigenschaften mechanischer, physikalischer oder chemischer Art durch Zersetzung in kleine Bruchstücke, die bei der Anwendung im physiologischen Bereich - insbesondere der Humananwendung - auch als Metabolite bezeichnet werden. Als Beispiel sei ein biologisch abbaubarer chirurgischer Nähfaden zitiert, der zunächst aufgrund seiner Festigkeit für den Zusammenhalt bei einer festgenähten Wunde sorgt, dann aber im Laufe der Zeit vom Körper abgebaut wird, wobei selbstverständlich die Abbaugeschwindigkeit und das Zusammenwachsen der Wunde in einem entsprechenden Verhältnis stehen müssen.

Als biologisches Milieu, in dem biologisch abbaubare Polymere eingesetzt werden können, sind solche Milieus zu betrachten, wie sie in der Natur vorkommen z. B. Wasser, Luft und Erde, jedoch auch der menschliche oder tierische Körper sowie das innere von Pflanzen. Vielfach werden biologisch abbaubare Polymere in einer Form verwendet, wo sie nur einem Zwecke dient, z. B. chirurgische Nähfäden, die lediglich die Funktion haben, für eine bestimmte Zeit die Wunde festzuhalten oder Müllbeutel oder Verpackungsfolien, die nach Benutzung biologisch abgebaut werden, wobei die Abbauprodukte selbst keine besondere Funktion ausüben.

Es ist aber auch möglich, den biologisch abbaubaren Polymeren Wirksubstanzen beizumengen oder die Polymere bereits bei der Synthese chemisch so zu gestalten, daß sie selbst neben ihrer mehr oder weniger mechanischen Funktlon auch andere Wirkfunktlon entwickeln. So können chirurgische Nähfäden hergestellt werden, die neben ihrer mechanischen Funktion beispielsweise eine desinfizierende Wirkung haben oder eine bestimmte medikamentöse Wirkung entfalten. Müllsäcke können beispielsweise Geruchsstoffe enthalten, die abstoßend auf Hunde und Katzen wirken.

Von Bedeutung bei biologisch abbaubaren Polymeren ist ferner, daß die Abbauprodukte weitgehend verträglich sind mit dem Milieu, in dem sie entstehen. So ist es selbstverständlich, daß biologisch abbaubare Polymere im medizinischen Bereich nur dann mit Erfolg eingesetzt werden können, wenn die bei dem biologischen Abbau entstehenden kleineren Bruchstücken (sog. Metabolite) unbedenklich sind.

Schließlich ist man auch sehr daran interessiert, die biologisch abbaubaren Polykondensate weitgehend aus Edukten zu gewinnen, die direkt in der Natur vorkommen oder aus Produkten gewonnen werden, die in der Natur vorkommen, d. h. aus sog. nachwachsenden Rohstoffen. Als molekulare Bausteine kommen, hier insbesondere Verbindungen in Frage, die im Stoffwechsel in der Natur, sei es im Menschen, im Tier oder in der Pflanze auftreten. Dazu gehören auch Verbindungen, welche durch Hydrolyse, Oxidation, Reduktion oder Abspaltung von Wasser aus Produkten wie Kohlehydraten gewonnen werden können. Zu diesen Verbindungen gehören auch die sog. Dianhydrohexite, welche durch Dehydratisierung der entsprechenden sechswertigen Alkohole erhalten werden, nämlich Isosorbit eine Verbindung, die durch Dehydrierung von Sorbit gewonnen wird und auch 1,4:3,6 Dianhydro-D-sorbit genannt wird (DAS), 1,4:3,6 Dianhydro-D-mannit (DAM), eine Verbindung die durch Dehydrierung von Mannit gewonnen wird und 1,4:3,6 Dianhydro-L-idit (DAI).

Es gibt bereits eine umfangreiche Literatur, vor allem wissenschaftliche Veröffentlichungen, die sich mit der Herstellung der Dianhydrohexite und deren Verwendung zur Herstellung von Polykondensaten befassen. Dabei ist noch darauf hinzuweisen, daß vor allem im deutschen Schrifttum die Schreibweisen für ein und dieselbe Verbindung unterschiedlich ist. Man findet u.a. die Bezeichnung Isosorbid, Isomannid und Isoiodid als auch 1,4:3,6-Dianhydro-D-sorbit (DAS) usw.

So beschreiben E.Flèche et al in starch/stärke 38 (1) 26-30 (1986) die Herstellung und Eigenschaften von Isosorbit. J. Thiem et al befassen sich in starch/stärke 36 (5) 170-6 (1984) mit der Darstellung und gezielten Polykondensation von Anhydroalditol-Bausteinen aus Stärke. Die Synthese von Polyestern aus DAS, DAM (1,4:3,6-Dianhydro-D-mannit) und DAJ (1,4:3,6-Dianhydro-L-idit) wird in Makromol Chem 194, 53-46, 1993 von Storbeck et al beschrieben. An weiteren Literaturstellen seien genannt Polymer 34 (23) 5003-6 (1993); Journal of Polymer Science: Part A: Polymer Chemistry 33 , 2813-20 (1995); Journal of Applied Polymer Science 59, 1199-1202 (1996); Die Angewandte Makromolekulare Chemie 199 (Nr. 3530) 191-205 (1992) und 210 (Nr. 3659) 173-196 (1993); J. Polym. Sci., Part A : Polym. Chem., 33, 2813 (1995); J. Appl. Polym. Sci., 62, 2257(1996).

In der DE-PS-1 263 981 werden modifizierte Polyester beschrieben, bei denen die Gfykolkomponente bis zu 20 Gew.-% aus Isosorbit bestehen kann und die weiterhin durch polyfunktionelle Säureester von drei bis fünfwertigen Säuren verzweigt sein können. Diese Polykondensate sind jedoch nicht biologisch abbaubar.

Polykondensate, die Amidfunktionen aufweisen, werden z.B. in Trends in Polymer Science 2(12) 425-36 (1994) und Journal of Polymer Science Part A: Polymer Chemistry 30, 2059-62 (1992) beschrieben.

Es sind somit bereits eine ganze Reihe von biologisch abbaubaren Polykondensaten bekannt, jedoch weisen diese die verschiedensten Nachteile auf. So ist es oft schwierig ein ausreichend hohes Mclekulargewicht einzustetten, andere Polykondensate setzen beim biologischen Abbau in zu großen Mengen schädliche Substanzen frei, andere wiederum sind aufgrund hoher Herstellungskosten nur bedingt zugänglich; darüber hinaus fehlt es an Polykondensaten, die ganz speziell für bestimmte Einsatzzwecke in ihren Eigenschaften eingestellt werden können.

Obwohl bereits eine Vielzahl von biologisch abbaubaren Polykondensaten bekannt sind, besteht noch ein Bedürfnis nach verbesserten Produkten, nach vorteilhaften Herstellungsverfahren und nach Produkten, die vielseitig verwendbar sind.

Aufgabe der Erfindung ist es somit, biologisch abbaubare Polykondensate zur Verfügung zu stellen, die ein gutes Abbauvermögen aufweisen, die sich einfach herstellen lassen und ganz oder teilweise aus nachwachsenden Rohstoffen hergestellt werden können und die bereits bei der Synthese so modifiziert werden können, daß sie für die vielfältigsten Einsatzzwecke geeignet sind.

Diese Aufgabe wird gelöst durch biologisch abbaubare Polykondensate gemäß Patentanspruch 1. In den Patentansprüchen 2 bis 7 werden besonders vorteilhafte Ausführungsformen der erfindungsgemäßen Polykondensate wiedergegeben. Die Herstellung dieser erfindungsgemäßen Polykondensate kann nach Verfahren gemäß den Patentansprüchen 8 bis 11 stattfinden. In den Ansprüchen 12 und 13 werden besonders vorteilhafte Verwendungsmöglichkeiten der erfindungsgemäßen Polykondensate wiedergegeben.

a) Die erfindungsgemäß eingesetzten Dianhydrohexite nämlich Isosorbit, Isomannit und Isoidit können nach an sich bekannten Verfahren durch Dehydratisierung der entsprechenden Hexite wie Sorbit (Sorbitol), Mannit (Mannitol) und andere hergestellt werden. Sie lassen sich wie z. B. das Isosorbit in größerem Maßstab aus Stärke gewinnen und sind im Handel erhältlich.

b) Die als zweite Komponente eingesetzten zweiwertigen organischen Carbonsäuren können aliphatischer, cycloaliphatischer oder aromatischer Art sein. Hier seien als Beispiele genannt Terephthalsäure, Adipinsäure, Furandicarbonsäure sowie die bevorzugt verwendete 3,6,9-Trioxaundecandicarbonsäure. Die Dicarbonsäuren können bei der Synthese als solche eingesetzt werden es ist aber auch möglich, entsprechende Derivate wie Säurechloride oder Ester der Carbonsäuren einzusetzen.

c) Als Beispiele für die mehrwertigen organischen Carbonsäuren, die außer zwei Carbonsäurefunktionen mindestens noch eine weitere gegebenenfalls gekappte, nämlich OH- und/oder COOH-Funktion aufweisen, seien erwähnt Weinsäure, Äpfelsäure, Oxybernsteinsäure, Zitronensäure, Isozitronensäure, Aconitsäure und ähnliche. Insbesondere können auch Cholsäure oder Desoxycholsäure verwendet werden, die sich durch ihre biologischen Funktionen für einen Einbau in funktionellen Biopolymeren eignen.

Zur Gruppe c) ist anzufügen, daß diese Carbonsäuren als solche eingesetzt werden können, d.h. bei denen die zwei oder mehr Funktionen nicht gekappt sind, also frei sind und der sofortigen Kondensationsreaktion zugänglich sind, sie können aber auch verkappt, z. B. veräthert oder verestert eingesetzt werden. Hierbei ist es durchaus möglich, daß von einigen trifunktionellen Bausteinen durchaus alle drei Funktionen an der Polyreaktion beteiligt sind. Daneben sind auch Teilreaktionen vorstellbar, bei denen nur die Reaktion einer funktionellen Gruppe zum Einbau in das Polymer führt. In dieser Funktion als sog. Polymerendgruppe nehmen die zwei weiteren funktionellen Gruppen nicht an der Polyreaktion teil. Auf jeden Fall können die nicht an der Polyreaktion teilnehmenden funktionellen Gruppen entweder weiterhin verkappt (geschützt) vorliegen, oder aber im Anschluß an die Polymerherstellung in einer polymeranalogen Reaktion die Verkappung aufgehoben werden. Dies entspricht chemisch der Abspaltung einer sogenannten Schutzgruppe, die zur Verkappung der Funktion zunächst eingeführt wurde.

Neben diesen drei Komponenten a), b) und c) können in die biologisch abbaubaren Polykondensate noch weitere Komponenten eingebaut werden, insbesondere zweiwertige Verbindungen wie alpha-Aminosäuren, z.B. Serin, Glutamin, Lysin, Glutaminsäure, Asparaginsäure, Cystein, es ist aber auch möglich, Hydroxycarbonsäuren oder Dihydroxyverbindungen mit zu verwenden. Durch den Einbau von derartigen Molekülen wird das stöchiometrische Verhältnis der zwei verschiedenen funktionellen Gruppen nicht verändert. Durch den Einbau von z. B. 4-Hydroxybenzoesäure werden statistischen Gesetzen folgend die Abstände zwischen gleichartigen Monomeren verlängert und damit auf dem Fachmann bekannte Weise gezielt Einfluß auf die polymerphysikalischen Eigenschaften, z. B. Glasübergangstemperaturen, genommen.

Die Polykondensation kann nach an sich üblichen Verfahren durchgeführt werden wie die sog. Eintropfreaktionen, bei denen sämtliche Ausgangsprodukte gleich zu Anfang gut vermischt werden und mit oder ohne Verwendung eines Katalysators zur Reaktion gebracht werden. Diese Reaktion kann in der Schmelze und auch in Lösung durchgeführt werden.

Es sind aber auch vollkontinuierliche oder semikontinuierliche Verfahren möglich.

Als Lösungsmittel sind neben dem bevorzugt verwendeten Dichlormethan u.a. auch Dimethylformamid, Dimethylsulfoxid und N-Methylpyrrolidon sehr geeignet.

Die Umsetzung wird zweckmäßig so durchgeführt, daß gleiche molare Mengen von OH-Gruppen mit gleichen molaren Mengen an COOH-Gruppen reagieren können. Es sind jedoch auch Abweichungen von den stöchiometrischen Verhältnissen möglich, vorzugweise beträgt die Abweichung vom stöchiometrischen Verhältnis jedoch nicht mehr als 5 Mol-%.

In der Regel ist es vorteilhaft darauf zu achten, daß eine Stöchiometrie der verschiedenen funktionellen Gruppen aufeinander abgestimmt ist. Im Idealfall sind z.B. Diolkomponenten und Dicarbonsäurekomponenten in äquimolaren Verhältnis vorhanden. Für bestimmte Anwendungen, z.B. wenn ein retardierter oder ein beschleunigter biologischer Abbau erforderlich ist, kann es zweckmäßig sein, von diesem Idealzustand abzuweichen. So ist es nach den dem Fachmann bekannten Methoden auch möglich, vernetzte Polymere (Gele) herzustellen.

Die Dianhydrohexite wie z.B. Isosorbid, welche gemäß der Erfindung zum Aufbau der Polykondensate eingesetzt werden, reagieren als Glykole mit den COOH-Gruppen der verwendeten Carbonsäuren. Vorteilhaft werden diese Verbindungen in molaren Mengen eingesetzt, daß mindestens 50 Prozent vorzugsweise mindestens 70 der COOH-Gruppen der Säuren mit den OH-Gruppen der Dianhydrohexite reagieren können.

Besonders vorteilhaft lassen sich die Eigenschaften der erfindungsgemäßen Polykondensate durch Einbau weiterer Bausteine modifizieren, so daß es möglich ist, maßgeschneiderte Polykondensate für spezielle Anwendungszwecke herzustellen. Hier ist insbesondere die Mitverwendung von Aminosäuren und Hydroxicarbonsäuren zu erwähnen, z.B. Hydroxibuttersäure, 4-Hydroxybenzoesäure, 2-Hydroxy-6-naphthoesäure, 4-Aminobenzoesäure, Glykolsäure, 4-Hydroxybiphenyl-4-carbonsäure, Lithocholsäure, 4-Hydroxyzimtsäure.

Die erfindungsgemäßen Polykondensate zeichnen sich durch eine sehr gute biologische Abbaubarkeit aus. Ohne sich hier auf eine Theorie festlegen zu wollen, wird vermutet, daß die Verbesserung der biologischen Abbaubarkeit gegenüber anderen biologisch abbaubaren Polykondensaten durch den Einbau von den Bausteinen mit den mindestens drei Funktionen bewirkt wird, d.h. durch die mehrwertige Carbonsäure, welche noch eine oder mehrere OH- oder COOH-Funktionen aufweisen, wie z.B. Apfelsäure.

Es ist ferner möglich, die Abbaubarkeit noch durch die Mitverwendung von kondensationsfähigen Edukten positiv zu beeinflussen, welche Heteroatome aufweisen. Hier ist besonders die 3,6,9-Trioxandecaundicarbonsäure zu nennen. Auch ist das Miteinbauen von Verbindungen, welche Aminofunktionen besitzen, vorteilhaft.

Unter biologisch abbaubar im Rahmen der Erfindung ist zu verstehen, daß das Polykondensat, wenn es in einem entsprechenden Milieu eingesetzt wird, im Laufe der Zeit in kleinere Spaltprodukte abgebaut wird, die vorzugsweise unbedenklich gegenüber der Umwelt Mensch, Tier und Pflanze sind. Unter biologisch abbaubar sind auch Begriffe zu verstehen wie bioerodierbar, enzymatisch spaltbar, kompostierbar, biodegradierbar, hydrolisierbar, verdaubar, eßbar usw.

Die hier erfindungsgemäß beschriebenen bioabbaubaren Polymere eignen sich aufgrund des Einbaus von Verbindungen mit mehr als zwei funktionellen Gruppen vor allem zum Einsatz als bioabbaubare Funktionspolymere.

Die Polykondensate gemäß der Erfindung lassen sich im schmelzflüssigen Zustand verarbeiten, sie können auch unter Verwendung entsprechender Lösungsmittel verarbeitet werden. Sie lassen sich zu allen möglichen Formkörpern wie Beutel, Folien, Tabletten, Kapseln, Fäden, Hohlfäden, Pulver u. dgl. verarbeiten.

Sie sind besonders dort verwendbar, wo es um die kontrollierte Abgabe von Wirkstoffen geht. Dabei kann der Wirkstoff bereits in das Polykondensat eingebaut sein; er wird dann bei dem biologischen Abbau im Medium selbst frei und kann dann seine Wirkung entfalten.

Es ist ferner möglich, aus den Polykondensaten gemäß der Erfindung Körper wie Kapseln herzustellen, die einen Wirkstoff umhüllen, der dann entweder nach einer gewissen Zeit, wenn die Kapsel aufgelöst ist, freigegeben wird, oder der vom Milieu im Laufe der Zeit aus der Kapsel herausgelaugt wird.

Es ist aber auch möglich, die Polykondensate als Matrix zu verwenden, d.h. der Wirkstoff wird in dem Polymer, z.B. in der Schmelze oder auch in einer Lösung gleichmäßig verteilt, so daß die Wirkstoffabgabe mit dem Abbau des Polykondensats einhergeht.

Die Polykondensate lassen sich auf den verschiedensten Gebieten verwenden. So kann man sie in der Landwirtschaft für die kontrollierte Abgabe von Düngemitteln, von Herbiziden, Pestiziden und dergl. einsetzen. Ein weites Einsatzgebiet ist der pharmazeutische Bereich, wo die Polykondensate z.B. als Tabletten eingesetzt werden können. Im medizinischen Bereich lassen sich die Polykondensate z.B. in entsprechender Form, etwa in Form von Mikropartikeln oder Extrudaten, auch subkutan einsetzen.

Das Herstellungsverfahren ist sehr variabel und steuerbar, so daß reproduzierbar auch unterschiedliche Molekulargewichte eingestellt werden können sowie Polykondensate mit maßgeschneidertem Eigenschaftsprofil.

Die Erfindung wird durch folgende Beispiele näher erläutert:

Beispiel 1

3,18 g (21,76mmol). L-Isosorbit, 2,472 g (10,88mmol) Dichlor-2,3-o-isopropyliden-Ltartrat, 2,209 g (10,88 mmol) Terephthalsäuredichlorid, 50ml getrocknetes Dichlormethan und 3,6 ml (44,60 mmcl) Pyridin werden gemischt und 3 Tage unter Rückfluß gerührt. Währenddessen bildet sich ein weißer Niederschlag. Anschließend wird das Polykondensat in Cyclohexan ausgefällt. Nach dem Trocknen in Tetrahydrofuran (THF) / Aceton gelöst und in Wasser ausgefällt, abgesaugt und im Vakuum getrocknet.

Weißes Pulver; Ausbeute 3,9 g entsprechen 64 % der Theorie.

| Elementaranalyse: | % C | % H |
|---|---|---|
| ber. | 56,3 | 4,9 |
| gef. | 56,8 | 5,3 |

Drehwertbestimmung gelöst in Dichlormethan

$$(\alpha)_D^{25} = + 82,4°$$

Molekulargewicht 6.700 ($M_w$) (nach vorangegangener Eichung einer Gelpermeationschromatography (GPC) mittels Polystyrolstandards)

Beispiel 2

3,046 g (20,8 mmol) L-Isosorbid, 1,15 g (6,28 mmol) Adipinsäuredichlorid, 3,31 g (14,57 mmol) Dichlor-2,3-o-isopropyliden-L-tartrat, 70 mml getrocknetes Dichlormethan und 3,3 ml (41,72 mmol) Pyridin werden gemischt. Sodann wird analog Beispiel 1 weiter verfahren.

Weißes Pulver; Ausbeute 3,8 g entsprechen 63 % der Theorie.

| Elementaranalyse: | C % | H % |
|---|---|---|
| ber. | 53,1 | 5,6 |
| gef. | 58,4 | 5,5 |

Drehwertbestimmung gelöst in Dichlormethan: $(\alpha)_D^{25} = + 78,6°$ Molekulargewicht 6.400 Dalton ($M_w$), Uneinheitlichkeit ($M_w/M_n = 2,9$) (nach vorangegangener Eichung einer Gelpermeationschromatography (GPC) mittels Polystyrolstandards)

Beispiel 3

3,193 g (21,85 mmol) L-Isosorbid 1,489 g (6,55 mmol) Dichlor-2,3-o-isopropyliden-L-tartrat, 2,952 g (15,30 mmol) Furandicarbonsäuredichlorid,
70 ml getrocknetes Dichlormethan und 3,5 ml (44,5 mmol) Pyridin werden gemischt. Sodann wird analog Beispiel 1 weiter verfahren.

Weiße Pulver; Ausbeute 4,9 g entsprechend 83 % der Theorie.

| Elementaranalyse: | C % | H % |
|---|---|---|
| ber. | 42,3 | 4,3 |
| gef. | 42,9 | 4,5 |

Drehwertbestimmung gelöst in Dichlormethan: $(\alpha)_D^{25} = -35,3°$ Molekulargewicht 8.200 Dalton ($M_w$) (nach vorangegangener Eichung einer Gelpermeationschormatography (GPC) mittels Polystyrolstandards)

Beispiel 4

Die Abbaubarkeit der Polymere wurde mittels eines modifizierten Sturm Tests untersucht. Der Text wurde gemäß der OECD-Richtlinie 301 B (1992; Sturm Test) durchgeführt. Der Test dient der Bestimmung der biologischen Abbaubarkeit organischer Substanzen durch aerobe Mikroorganismen in einem wäßrigen Testmedium. Die organische Prüfsubstanz ist in dem Experiment die einzige Kohlenstoffquelle und damit Energielieferant für die Mikroorganismen. Der Abbau der zu untersuchenden organischen Substanzen wird dabei in indirekter Form über die Messung des freigesetzten Kohlendioxids bestimmt. Die Testdauer beträgt jeweils 28 Tage. Als innerer Standard dient Natriumbenzoat.

Die Prüfbedingungen sind im einzelnen: Verwendung eines Überstands vom Belebtschlamm der Kläranalage Frankfurt Niederrad (10 ml pro 1 l Prüfansatz); Braunglasflaschen mit 5 l Füllvolumen; Magnetrührer; Raumtemperatur; $CO_2$-Bestimmung über Bariumcarbonat-Bildung in wässriger $Ba(OH)_2$-Lösung und Rücktitration der überschüssigen Lauge.

| ZEIT (TAGE) | BLINDANSATZ $CO_2$ (mg) | NATRIUM BENZOAT $CO_2$ (mg) | NATRIUM BENZOAT ABBAUGRAD (%) | POLY-L-MILCHSÄURE ABBAUGRAD (%) VERGLEICHS-BEISPIEL |
|---|---|---|---|---|
| 1 | 0,7 | 1,6 | 1 | 2 |
| 4 | 2,4 | 24,3 | 34 | 8 |
| 7 | 3,6 | 40,7 | 58 | 18 |
| 11 | 4,6 | 50,7 | 72 | 38 |
| 14 | 5,3 | 55,6 | 78 | 53 |
| 18 | 6,7 | 59,3 | 81 | 64 |
| 21 | 8,4 | 61,9 | 83 | 71 |
| 25 | 10,7 | 65,4 | 85 | 79 |
| 28 | 14,8 | 69,6 | 85 | 82 |

| ZEIT (TAGE) | NATRIUM BENZOAT ABBAUGRAD (%) | POLYMER BEISPIEL 1 ABBAUGRAD (%) | POLYMER BEISPIEL 2 ABBAUGRAD (%) | POLYMER BEISPIEL 3 ABBAUGRAD (%) |
|---|---|---|---|---|
| 1 | 1 | 1 | 2 | 2 |
| 4 | 34 | 2 | 14 | 8 |
| 7 | 58 | 20 | 29 | 26 |
| 11 | 72 | 51 | 58 | 45 |
| 14 | 78 | 66 | 75 | 61 |
| 18 | 81 | 79 | 79 | 74 |
| 21 | 83 | 83 | 83 | 78 |
| 25 | 85 | 86 | 85 | 82 |
| 28 | 85 | 89 | 87 | 85 |

**Patentansprüche**

1. Nach OECD-Richtline 301 B biologisch abbaubare Polykondensate, die sich von mindestens drei Komponenten ableiten, **dadurch gekennzeichnet, dass** aus jeder der drei Gruppen der

   a) Dianhydrohexite
   b) zweiwertige organische Carbonsäuren
   c) mehrwertige organische Carbonsäuren, die außer zwei Carbonsäurefunktionen mindestens eine weitere, gegebenenfalls gekappte Funktion aufweisen, nämlich -OH und/oder -COOH.

   jeweils mindestens eine Komponente ausgewählt ist.

2. Biologisch abbaubare Polykondensate nach Anspruch 1 **dadurch gekennzeichnet, daß** die zweiwertige organische Carbonsäure Terephthalsäure ist.

3. Biologisch abbaubare Polykondensate nach Anspruch 1 **dadurch gekennzeichnet, daß** die zweiwertige organische Carbonsäure 3,6,9-Trioxaundecandicarbonsäure ist.

**4.** Biologisch abbaubare Polykondensate nach Anspruch 1 **dadurch gekennzeichnet, daß** die zweiwertige organische Carbonsäure Furandicarbonsäure ist.

**5.** Biologisch abbaubare Polykondensate nach mindestens einem der Ansprüche 1 bis 4 **dadurch gekennzeichnet, daß** die mehrwertige organische Carbonsäure nach c) Weinsäure ist.

**6.** Biologisch abbaubare Polykondensate nach mindestens einem der Ansprüche 1 bis 4 **dadurch gekennzeichnet, daß** die mehrwertige organische Carbonsäure nach c) Traubensäure ist.

**7.** Biologisch abbaubare Polykondensate nach mindestens einem der Ansprüche 1 bis 4, **dadurch gekennzeichnet, daß** die mehrwertige organische Carbonsäure nach c) Zitronensäure ist.

**8.** Verfahren zur Herstellung nach biologisch abbaubaren Polykondensaten nach einem der Ansprüche 1 bis 7 **dadurch gekennzeichnet, daß** die Edukte in der Schmelze umgesetzt werden.

**9.** Verfahren zur Herstellung biologisch abbaubarer Polykondensate nach einem der Ansprüche 1 bis 7 **dadurch gekennzeichnet, daß** die Edukte in einem Lösungsmittel umgesetzt werden.

**10.** Verfahren nach Anspruch 9 **dadurch gekennzeichnet, daß** man als Lösungsmittel Dichlormethan verwendet.

**11.** Verfahren zur Herstellung biologisch abbaubarer Polykondensate nach einem der Ansprüche 8 bis 10 **dadurch gekennzeichnet, daß** man die Edukte bezüglich der OH- und COOH-Funktionen in equimolarem Verhältnis $\pm$ 5 Mol-% umsetzt.

**12.** Verwendung der biologisch abbaubaren Polykondensate nach einem der Ansprüche 1 bis 7 sowie hergestellt nach einem Verfahren gemäß einem der Ansprüche 8 bis 11 als Verkapselungsmaterial für Wirkstoffe.

**13.** Verwendung der biologisch abbaubaren Polykondensate nach einem der Ansprüche 1 bis 7 bzw. hergestellt nach einem Verfahren nach einem der Ansprüche 8 bis 11 als Matrixmaterial zur Aufnahme von Wirkstoffen.

**Claims**

**1.** Polycondensates being biodegradable according to the OECD Directive 301 B, and which derive from at least three components, **characterized in that** from each of the three groups

    a) dianhydrohexitols
    b) dibasic organic carboxylic acids, and
    c) polyfunctional organic carboxylic acids which, besides two carboxylic acid functions, have at least one other uncapped or capped function, specifically OH and/or COOH, respectively at least one component is selected.

**2.** A biodegradable polycondensate as claimed in claim 1, wherein the dibasic organic carboxylic acid is terephthalic acid.

**3.** A biodegradable polycondensate as claimed in claim 1, wherein the dibasic organic carboxylic acid is 3,6,9-trioxaundecanedicarboxytic acid.

**4.** A biodegradable polycondensate as claimed in claim 1, wherein the dibasic organic carboxylic acid is furan dicarboxylic acid.

**5.** A biodegradable polycondensate as claimed in at least one of claims 1 to 4, wherein the polyfunctional organic carboxylic acid of c) is tartaric acid.

**6.** A biodegradable polycondensate as claimed in at least one of claims 1 to 4, wherein the polyfunctional organic carboxylic acid of c) is uvic acid.

**7.** A biodegradable polycondensate as claimed in at least one of claims 1 to 4, wherein the polyfunctional organic carboxylic acid of c) is citric acid.

8. A process for preparing biodegradable polycondensates as claimed in any of claims 1 to 7, which comprises reacting the starting materials in the melt.

9. The process for preparing biodegradable polycondensates as claimed in any of claims 1 to 7, wherein the starting materials are reacted in a solvent.

10. The process as claimed in claim 9, wherein dichloromethane is used as solvent.

11. The process for preparing biodegradable polycondensates as claimed in any of claims 8 to 10, wherein the ratio in which the starting materials are reacted, based on the OH and COOH functions, is equimolar ± 5 mol%.

12. The use of the biodegradable polycondensates as claimed in any of claims 1 to 7, or else prepared by a process as claimed in any of claims 8 to 11, as an encapsulating material for active substances.

13. The use of the biodegradable polycondensates as claimed in any of claims 1 to 7, or prepared by a process as claimed in any of claims 8 to 11, as a matrix material for receiving active substances.


**Revendications**

1. Polycondensats biodégradables selon la Directive 301 B de l'OCDE, qui dérivent d'au moins trois composants, **caractérisés en ce que** chaque fois au moins un composant est pris dans chacun des trois groupes

   a) dianhydrohexites,
   b) acides carboxyliques organiques bivalents,
   c) acides carboxyliques organiques multivalents qui présentent en dehors de deux fonctions acide carboxylique, au moins une autre fonction, éventuellement coiffée, à savoir -OH et/ou -COOH.

2. Polycondensats biodégradables selon la revendication 1, **caractérisés en ce que** l'acide carboxylique organique bivalent est l'acide téréphtalique.

3. Polycondensats biodégradables selon la revendication 1, **caractérisés en ce que** l'acide carboxylique organique bivalent est l'acide 3,6,9-trioxaundécanedicarboxylique.

4. Polycondensats biodégradables selon la revendication 1, **caractérisés en ce que** l'acide carboxylique organique bivalent est l'acide furannedicarboxylique.

5. Polycondensats biodégradables selon au moins l'une des revendications 1 à 4, **caractérisés en ce que** l'acide carboxylique organique multivalent selon c) est l'acide tartrique.

6. Polycondensats biodégradables selon au moins l'une des revendications 1 à 4, **caractérisés en ce que** l'acide carboxylique organique multivalent selon c) est l'acide racémique.

7. Polycondensats biodégradables selon au moins l'une des revendications 1 à 4, **caractérisés en ce que** l'acide carboxylique organique multivalent selon c) est l'acide citrique.

8. Procédé pour la préparation de polycondensats biodégradables selon l'une des revendications 1 à 7, **caractérisé en ce qu'**on fait réagir les produits de départ en fusion.

9. Procédé pour la préparation de polycondensats biodégradables selon l'une des revendications 1 à 7, **caractérisé en ce qu'**on fait réagir les produits de départ dans un solvant.

10. Procédé selon la revendication 9, **caractérisé en ce qu'**on utilise le dichlorométhane comme solvant.

11. Procédé pour la préparation de polycondensats biodégradables selon l'une des revendications 8 à 10, **caractérisé en ce qu'**on fait réagir les produits de départ dans un rapport molaire de ±5 % molaires par rapport aux fonctions -OH et -COOH.

**12.** Utilisation des polycondensats biodégradables selon l'une des revendications 1 à 7, ainsi que préparés selon un procédé selon l'une des revendications 8 à 11, en tant que matière d'encapsulation de principes actifs.

**13.** Utilisation des polycondensats biodégradables selon l'une des revendications 1 à 7, respectivement préparés selon un procédé selon l'une des revendications 8 à 11, en tant que matière de matrice pour absorption de principes actifs.